# EUROPÄISCHE PATENTSCHRIFT

(11) **EP 0 654 254 B1**
(45) Veröffentlichungstag und Bekanntmachung des Hinweises auf die Patenterteilung: **08.09.1999**
(21) Anmeldenummer: 94115332.2
(22) Anmeldetag: 29.09.1994
(51) Int. Cl.: A61F 2/32, A61F 2/38, A61F 2/68

(54) **Prothesengelenk**
Prosthetic joint
Prothèse d'articulation

(30) Priorität: 15.11.1993 DE 4338946
(43) Veröffentlichungstag der Anmeldung: 24.05.1995
(73) Patentinhaber: Otto Bock Orthopädische Industrie Besitz- und Verwaltungs-Kommanditgesellschaft, 37115 Duderstadt (DE)
(72) Erfinder: Krukenberg, Manfred, D-37115 Duderstadt (DE)
(74) Vertreter: Gramm, Werner, Prof. Dipl.-Ing.

(56) Entgegenhaltungen:
- EP-A- 0 549 855
- DE-A- 2 101 303
- GB-A- 762 695
- GB-A- 2 264 348

## Beschreibung

Die Erfindung betrifft ein Prothesengelenk, insbesondere Knie- oder Hüftgelenk, mit einem Gelenkoberteil, einem Gelenkunterteil, einer diese beiden Gelenkteile verschwenkbar miteinander verbindenden, drehfest mit einem Gelenkteil verbundenen Gelenkachse und einer Dämpfung der Gelenkverschwenkung, wobei die Dämpfung in eines der beiden Gelenkteile integriert ist, unmittelbar die mit dem Gelenkteil drehfest verbundene Gelenkachse beaufschlagt und eine geschlossene, die Gelenkachse über ihren Umfang zumindest teilweise konzentrisch umschließende Verdrängerkammer aufweist, die von der als Drehkolben ausgebildeten Gelenkachse in zwei Teilkammern unterteilt ist.

Eine derartige Ausführungsform läßt sich der GB-A-762 695 entnehmen. Offenbart sind Ausführungsformen, die der Bedämpfung eines vordefinierbaren Teilbereichs lediglich der Beugebewegung dienen. Die übrige Beugebewegung sowie die vollständige Streckbewegung sollen hingegen weitgehend dämpfungsfrei erfolgen können. Die zwei Teilkammern der Verdrängerkammer sind über einen Bypass miteinander verbindbar, der einen Durchfluß in beiden Richtungen zuläßt, und dessen Durchflußöffnung mittels eines von außen verstellbaren Nadelventils manuell voreinstellbar ist.

Gekoppelt hiermit ist ein Bypass-System, das mit dem erstgenannten Bypass gekoppelt ist und über ein Kugelventil verfügt, das normalerweise durch eine Feder geschlossen gehalten wird. In dieser Stellung erfolgt der Durchfluß über den drosselbaren Bypass. Die Öffnung des Kugelventils erfolgt durch einen parallel zur Gelenkachse angeordneten Stößel, der durch seitliche Nocken an einer drehfest mit der Gelenkachse verbundenen Steuerscheibe betätigt wird. In der geöffneten Stellung des Kugelventils ist der Durchfluß weitgehend frei. Auf diese Weise läßt sich allein durch entsprechende Anordnung der seitlichen Nocken auf der Steuerscheibe bestimmen, in welchem Bereich der Gelenkverschwenkung Beugewiderstand erzeugt werden soll. Die Streckbewegung wird bei dieser Ausführungsform immer durch Öffnen des im Steuerblock vorgesehenen Rückschlagventils freigegeben.

Der Erfindung liegt die Aufgabe zugrunde, diese vorbekannte Ausführungsform hinsichtlich ihrer Funktion zu verbessern, indem zusätzlich eine Schwungphasensteuerung ermöglicht wird, bei der der Verschwenkungswiderstand hinsichtlich Flexion und Extension getrennt voneinander justiert werden kann.

Ausgehend von dem eingangs beschriebenen Prothesengelenk wird diese Aufgabe erfindungsgemäß dadurch gelöst, daß die Teilkammern der Verdrängerkammer über zwei parallel geschaltete, entgegengesetzt wirkende Drosselrückschlagventile miteinander verbunden und beide Drosselstellen separat voneinander von außen ansteuerbar sind. Bei dieser Ausführungsform läßt sich der Verschwenkungswiderstand hinsichtlich Flexion und Extension getrennt voneinander justieren.

Die Ansteuerung der Drosseln kann grundsätzlich mechanisch oder durch elektrische Stelltriebe erfolgen. Erfindungsgemäß ist es vorteilhaft, wenn die Ansteuerung einer Drossel durch eine axial verschiebbare Drosselstange erfolgt, deren Verschiebung beispielsweise durch eine manuell verstellbare Rändelschraube oder dergleichen vorgenommen werden kann.

Insbesondere bei einem als Kniegelenk ausgebildeten Prothesengelenk ist es vorteilhaft, wenn eine das Gelenk belastende Kraft ein vordefiniertes Schließen der Beugedrossel bewirkt. Dies läßt sich konstruktiv gestalten in Form einer Teleskop-, Kraft- oder Momentsteuerung.

Der Drehkolben weist vorzugsweise eine rechteckige Kolbenplatte auf, die an den beiden Stirnseiten und der zylindrischen Innenwandung der geschlossen ausgebildeten Verdrängerkammer über einen Dichtrahmen anliegt. Dieser kann vorzugsweise durch ein mit der Kolbenplatte verschraubtes Haltestück festlegbar sein. Man erhält dadurch eine allseits geschlossene Verdrängerkammer mit einem einzigen, im Bereich der Drehkolbendichtung vorgesehenen definierten Leck. Dabei ist es vorteilhaft, wenn die die Verdrängerkammer nach außen abdichtende Drehkolbendichtung außerhalb der Drehkolbenlagerung liegt. Die Schmierung der Drehkolbenlagerung erfolgt aufgrund des in der Verdrängerkammer herrschenden Überdrucks, also durch das Hydrauliköl, das deshalb hohe Schmiereigenschaften aufweisen sollte.

Weitere Merkmale der Erfindung sind Gegenstand der Unteransprüche und werden in Verbindung mit weiteren Vorteilen der Erfindung anhand eines Ausführungsbeispieles näher erläutert.

In der Zeichnung ist eine als Beispiel dienende Ausführungsform der Erfindung dargestellt. Es zeigen:

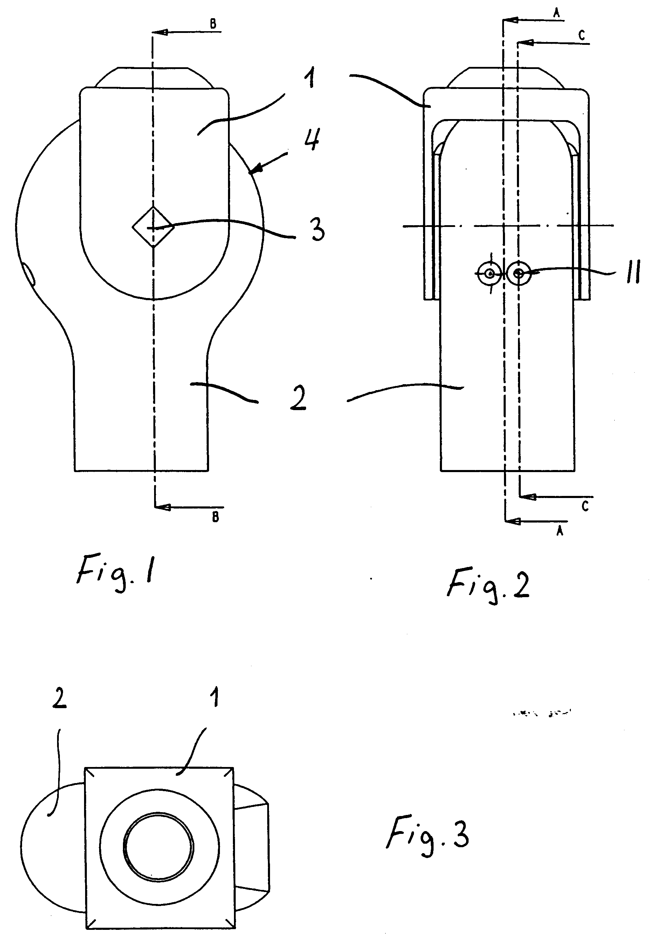

- **Figur 1 -**: ein Kniegelenk in Seitenansicht;

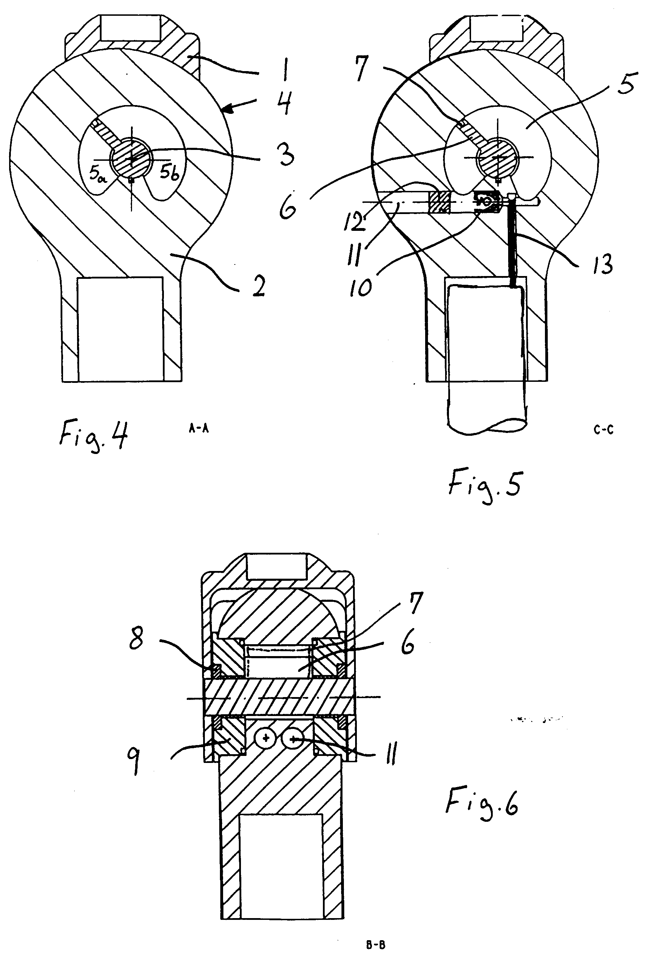

- **Figur 2 -**: die Darstellung gemäß **Figur 1** in Stirnansicht;
- **Figur 3 -**: die Darstellung gemäß **Figur 1** in Draufsicht;
- **Figur 4 -**: einen Längsschnitt gemäß der Linie A-A in **Figur 2**;
- **Figur 5 -**: einen Längsschnitt gemäß der Linie C-C in **Figur 2** und
- **Figur 6 -**: einen Querschnitt gemäß der Linie B-B in **Figur 1**.

Als Beispiel für ein Prothesengelenk ist ein Kniegelenk dargestellt, das aus einem Gelenkoberteil 1 und einem etwas exzentrisch hierzu angeordneten Gelenkunterteil 2 besteht. Die verschwenkbare Verbindung beider Gelenkteile 1,2 erfolgt über eine drehfest mit dem Gelenkoberteil 1 verbundene Gelenkachse 3. Dabei stützt sich das Gelenkoberteil 1 mit seiner teilzylindrisch ausgebildeten Unterseite auf der zylindrischen Oberfläche 4 des Gelenkunterteils 2 ab. Aufgrund der exzentrischen Anordnung zueinander läuft bei einer Gelenkverschwenkung das Gelenkoberteil 1 mit seiner Unterseite auf die zylindrische Oberfläche 4 auf, die somit ganzflächig als Gelenkanschlag dient. Ein separater Gelenkanschlag kann daher entfallen.

Zur Dämpfung der Gelenkverschwenkung ist in dem dargestellten Ausführungsbeispiel eine unmittelbar in das Gelenkunterteil 2 integrierte hydraulische Dämpfung vorgesehen, die direkt auf die Gelenkachse 3 wirkt. Die hydraulische Dämpfung umfaßt eine geschlossene, die Gelenkachse 3 über ihren Umfang teilweise konzentrisch umschließende Verdrängerkammer 5, die von der als Drehkolben ausgebildeten Gelenkachse 3 in zwei Teilkammern 5a,5b unterteilt ist. Hierzu ist die Gelenkachse 3 mit einer rechteckigen, sich in axialer und radialer Richtung erstreckenden Kolbenplatte 6 bestückt, die mit ihren beiden radial verlaufenden Stirnrändern an den beiden Stirnwandungen der Verdrängerkammer 5 und mit ihrem axial verlaufenden Längsrand an der zylindrischen Innenwandung der Verdrängerkammer 5 abdichtend anliegt. Diese Abdichtung erfolgt vorzugsweise über einen Dichtrahmen 7, der durch ein mit der Kolbenplatte 6 verschraubtes in der Zeichnung nicht näher dargestelltes Haltestück auf der Kolbenplatte 6 befestigt ist.

Die Verdrängerkammer 5 ist nach außen durch eine Drehkolbendichtung 8 abgedichtet, die außerhalb der Drehkolbenlagerung 9 liegt. Die Schmierung erfolgt durch in der Verdrängerkammer 5 herrschenden Überdruck, wobei die Verdrängerkammer 5 vorzugsweise mit einem Hydrauliköl mit hohen Schmiereigenschaften befüllt ist.

Die Teilkammern 5a,5b sind über zwei parallel geschaltete, entgegengesetzt wirkende Drosselrückschlagventile 10 miteinander verbunden. Letztere sind in zwei im Gelenkunterteil 2 nebeneinanderliegenden Bohrungen 11 angeordnet, die senkrecht zur Gelenkachse 3 verlaufen und nach außen durch jeweils einen Dichtungsstopfen 12 verschlossen sind.

Zur Ansteuerung der Drosselstelle jedes Drosselrückschlagventils 10 ist in dem dargestellten Ausführungsbeispiel jeweils eine Drosselstange 13 vorgesehen, die durch eine nicht näher dargestellte Justiereinrichtung, beispielsweise eine manuell betätigbare Rändelschraube, längsverschiebbar ist und mit ihrem in Figur 5 dargestellten oberen freien Ende mehr oder weniger tief in den Strömungsquerschnitt der Strömungsverbindung zwischen den beiden Teilkammern 5a und 5b ragt. Durch die mögliche separate Verstellung der beiden Drosselstangen 13 läßt sich der Verschwenkungswiderstand getrennt justieren für Flexion und Extension. Dabei ist es zusätzlich vorteilhaft, wenn eine Belastung des Knies zu einem Schließen der Beugedrossel führt. Dies wird realisiert durch eine in der Zeichnung nicht näher dargestellte Teleskop-, Kraft- oder Momentsteuerung.

## Patentansprüche

1. Prothesengelenk, insbesondere Knie- oder Hüftgelenk, mit einem Gelenkoberteil (1), einem Gelenkunterteil (2), einer diese beiden Gelenkteile (1,2) verschwenkbar miteinander verbindenden, drehfest mit einem Gelenkteil (1) verbundenen Gelenkachse (3) und einer Dämpfung der Gelenkverschwenkung, wobei die Dämpfung in eines (2) der beiden Gelenkteile (1,2) integriert ist, unmittelbar die mit dem Gelenkteil (1) drehfest verbundene Gelenkachse (3) beaufschlagt und eine geschlossene, die Gelenkachse (3) über ihren Umfang zumindest teilweise konzentrisch umschließende Verdrängerkammer (5) aufweist, die von der als Drehkolben (3,6,7) ausgebildeten Gelenkachse (3) in zwei Teilkammern (5a,5b) unterteilt ist, **dadurch gekennzeichnet**, daß die Teilkammern (5a,5b) der Verdrängerkammer (5) über zwei parallel geschaltete, entgegengesetzt wirkende Drosselrückschlagventile (10) miteinander verbunden und beide Drosselstellen separat voneinander von außen ansteuerbar sind.

2. Prothesengelenk nach Anspruch 1, **dadurch gekennzeichnet**, daß die Ansteuerung einer Drossel durch eine axial verschiebbare Drosselstange (13) erfolgt.

3. Prothesengelenk nach Anspruch 2, **dadurch gekennzeichnet**, daß zur Verschiebung der Drosselstange (13) eine manuell verstellbare Rändelschraube oder dergleichen vorgesehen ist.

4. Prothesengelenk nach Anspruch 1,2 oder 3, **dadurch gekennzeichnet**, daß Mittel vorhanden sind, die es erlauben, daß eine das Gelenk belastende Kraft ein vordefiniertes Schließen der Beugedrossel bewirkt.

5. Prothesengelenk nach einem der vorhergehenden Ansprüche, **dadurch gekennzeichnet**, daß der Drehkolben (3,6,7) eine rechteckige Kolbenplatte (6) aufweist, die an den beiden Stirnseiten und der zylindrischen Innenwandung der Verdrängerkammer (5) über einen Dichtrahmen (7) anliegt.

6. Prothesengelenk nach Anspruch 5, **dadurch gekennzeichnet**, daß der Dichtrahmen (7) durch ein mit der Kolbenplatte (6) verschraubtes Haltestück festgelegt ist.

7. Prothesengelenk nach einem der vorhergehenden Ansprüche, **dadurch gekennzeichnet**, daß die die Verdrängerkammer (5) nach außen abdichtende Drehkolbendichtung (8) außerhalb der Drehkolbenlagerung (9) liegt.

8. Prothesengelenk nach einem der vorhergehenden Ansprüche, **dadurch gekennzeichnet**, daß zur Montage der beiden Drosselrückschlagventile (10) in dem die Verdrängerkammer (5) aufnehmenden Gelenkteil (2) zwei Bohrungen (11) vorgesehen sind, die nach außen durch einen Dichtungsstopfen (12) verschlossen sind.

9. Prothesengelenk nach einem der vorhergehenden Ansprüche, **dadurch gekennzeichnet**, daß das Dämpfungsmedium ein Hydrauliköl mit hohen Schmiereigenschaften ist.

10. Prothesengelenk nach einem der vorhergehenden Ansprüche, **dadurch gekennzeichnet**, daß sich das Gelenkoberteil (1) mit seiner teilzylindrisch ausgebildeten Unterseite auf der zylindrischen Oberfläche (4) des exzentrisch zum Gelenkoberteil angeordneten Gelenkunterteils (2) abstützt.

## Claims

1. A prosthesis joint, more particularly a knee or hip joint, with a joint upper section (1), a joint lower section (2), a joint axle (3) pivotably connecting these two joint sections (1, 2) with one another and non-rotatably connected to a joint section (1), and with a damping of the joint pivoting, the damping being integrated into one (2) of the two joint sections (1, 2), acting directly upon the joint axle (3) non-rotatably connected to the joint section (1) and comprising a closed displacement chamber (5), which at least partially concentrically encloses the joint axle (3) over its circumference and is divided into two partial chambers (5a, 5b) by the joint axle (3) constructed as a rotary piston (3, 6, 7), characterised in that the partial chambers (5a, 5b) of the displacement chamber (5) are connected to one another via two non-return throttle valves (10), which are connected in parallel and act in opposite directions, and both throttle sites can be externally actuated independently of one another.

2. A prosthesis joint according to claim 1, characterised in that the actuation of a throttle is effected by an axially displaceable throttle rod (13).

3. A prosthesis joint according to claim 2, characterised in that a manually adjustable knurled-head screw or the like is provided for the displacement of the throttle rod (13).

4. A prosthesis joint according to claim 1, 2 or 3, characterised in that means are provided, which allow a force loading the joint to effect a pre-defined closure of the bending throttle.

5. A prosthesis joint according to one of the preceding claims, characterised in that the rotary piston (3, 6, 7) comprises a rectangular piston plate (6), which rests against the two end faces and the cylindrical inner wall of the displacement chamber (5) via a sealing frame (7).

6. A prosthesis joint according to claim 5, characterised in that the sealing frame (7) is fixed in position by a retaining element screwed to the piston plate (6).

7. A prosthesis joint according to one of the preceding claims, characterised in that the rotary piston seal (8) sealing the displacement chamber (5) relative to the outside lies outside the rotary piston bearing (9).

8. A prosthesis joint according to one of the preceding claims, characterised in that two bores (11) are provided for the assembly of the two non-return throttle valves (10) in the joint section (2) accommodating the displacement chamber (5), the bores (11) being sealed relative to the outside by a sealing plug (12).

9. A prosthesis joint according to one of the preceding claims, characterised in that the damping medium is a hydraulic oil having high lubrication properties.

10. A prosthesis joint according to one of the preceding claims, characterised in that the joint upper section (1) is supported with its part-cylindrical underside upon the cylindrical surface (4) of the joint lower section (2) arranged eccentric to the joint upper section.

## Revendications

1. Articulation prothétique, en particulier articulation du genou ou de la hanche, comprenant une partie supérieure (1) de l'articulation, une partie inférieure (2) de l'articulation, un axe articulaire (3) solidaire en rotation de l'une (1) des parties d'articulation, et reliant ces deux parties d'articulation (1, 2) l'une avec l'autre de manière pivotante, et un amortissement du pivotement articulaire, lequel amortissement est intégré dans l'une (2) des deux parties d'articulation (1, 2), sollicite directement la partie d'articulation (1) qui est solidaire en rotation de l'axe articulaire (3), et présente une chambre volumétrique (5) fermée entourant concentriquement, au moins partiellement, l'axe articulaire (3) sur sa périphérie, la chambre volumétrique étant subdivisée en deux chambres partielles (5a, 5b) par l'axe articulaire (3) réalisé sous la forme d'un piston rotatif (3, 6, 7), caractérisée en ce que les chambres partielles (5a, 5b) de la chambre volumétrique (5) sont reliées l'une à l'autre par deux clapets anti-retour (10) formant étranglement, montés en parallèle et agissant en opposition, et en ce que les deux points d'étranglement sont réglables de l'extérieur séparément l'un de l'autre.

2. Articulation prothétique selon la revendication 1, caractérisée en ce que le réglage d'un étranglement s'effectue par une tige d'étranglement (13) montée à coulissement axial.

3. Articulation prothétique selon la revendication 2, caractérisée en ce que pour le coulissement de la tige d'étranglement (13), il est prévu une vis moletée réglable manuellement ou analogue.

4. Articulation prothétique selon la revendication 1, 2 ou 3, caractérisée en ce qu'il est prévu des moyens permettant qu'une force chargeant l'articulation produise une fermeture prédéfinie de l'étranglement de flexion.

5. Articulation prothétique selon l'une des revendications précédentes, caractérisée en ce que le piston rotatif (3, 6, 7) présente une plaque de piston rectangulaire (6) qui est en contact par un cadre d'étanchéité (7) avec les deux côtés frontaux et la paroi intérieure cylindrique de la chambre volumétrique (5).

6. Articulation prothétique selon la revendication 5, caractérisée en ce que le cadre d'étanchéité (7) est fixé par une pièce de maintien vissée avec la plaque de piston (6).

7. Articulation prothétique selon l'une des revendications précédentes, caractérisée en ce que l'étanchéité (8) du piston rotatif, assurant l'étanchéité de la chambre volumétrique (5) vers l'extérieur se trouve en-dehors de l'agencement de palier (9) du piston rotatif.

8. Articulation prothétique selon l'une des revendications précédentes, caractérisée en ce que pour le montage des deux clapets anti-retour à étranglement (10) dans la partie d'articulation (2) recevant la chambre volumétrique (5), il est prévu deux alésages (11) qui sont fermés vers l'extérieur par un bouchon d'étanchéité (12).

9. Articulation prothétique selon l'une des revendications précédentes, caractérisée en ce que le médium d'amortissement est une huile hydraulique à bonnes propriétés de lubrification.

10. Articulation prothétique selon l'une des revendications précédentes, caractérisée en ce que la partie supérieure (1) de l'articulation s'appuie, par sa face inférieure réalisée en cylindre partiel, sur la surface cylindrique (4) de la partie inférieure (2) de l'articulation, disposée excentrée par rapport à la partie supérieure de l'articulation.
